# EUROPÄISCHE PATENTSCHRIFT

(11) **EP 2 918 297 B1**
(45) Veröffentlichungstag und Bekanntmachung des Hinweises auf die Patenterteilung: **16.11.2016**
(21) Anmeldenummer: 14159377.2
(22) Anmeldetag: 13.03.2014
(51) Int. Cl.: A61M 1/00, A61F 9/007

(54) **Kassettenmodul**
Cartridge module
Module à cassettes

(43) Veröffentlichungstag der Anmeldung: 16.09.2015
(73) Patentinhaber: Oertli-Instrumente AG, 9442 Berneck (CH)
(72) Erfinder: Wangler, Christoph, 9404 Rorschacherberg (CH)
(74) Vertreter: Frischknecht, Harry Ralph

(56) Entgegenhaltungen:
- WO-A1-2008/060958
- WO-A1-2010/054145
- WO-A1-2010/056972
- US-A- 5 499 969
- US-A1- 2008 114 301
- US-B1- 7 833 206

## Beschreibung

### TECHNISCHES GEBIET

Die vorliegende Erfindung betrifft ein Kassettenmodul, insbesondere ein opthalmologisches Kassettenmodul, zur Aufnahme von abgesogenem Material, insbesondere Flüssigkeiten, nach dem Oberbegriff von Anspruch 1. Weiter betrifft die Erfindung ein Absaugsystem nach dem Oberbegriff von Anspruch 13 mit dem Kassettenmodul nach Anspruch 1.

### STAND DER TECHNIK

Aus dem Stand der Technik sind bei Operationsgeräten für die Augenchirurgie verschiedene Typen von Abflussbehältern zum Auffangen von aus dem Auge abgesaugtem Material bekannt. Diese sind abhängig von der verwendeten Vakuumpumpentechnologie unterschiedlich aufgebaut.

Bei Geräten mit Vakuumpumpen nach dem Venturi-Prinzip herrscht üblicherweise im Abflussbehälter ein Vakuum von bis zu 700mm Hg, wodurch dieser nicht nur der Aufnahme der abgesaugten Materialien dient, sondern auch als Vakuumbehälter. Der Abflussbehälter muss in diesem Fall formstabil und dickwandig ausgeführt sein, so dass der Behälter als Vakuumbehälter bei diesen Druckdifferenzen einsetzbar ist. Aufgrund dieser Ausführung wird bei der Herstellung der Abflussbehälter vergleichsweise viel Material benötigt, was sich im Preis sowohl bei der Anschaffung als auch bei der Entsorgung negativ ausdrückt. Zudem ist der Materialbedarf bei Einweg-Abflussbehältern, die aufgrund der höheren Patientensicherheit gegen Infektionen und einfacherer Abläufe im Operationssaal immer mehr eingesetzt werden, relativ gross.

Weiter ist auch der Herstellaufwand als solches relativ hoch, weil die Abflussbehälter üblicherweise aus mehreren Teilen, die über Ultraschallschweissen miteinander verbunden werden, hergestellt werden. Dies erhöht die Kosten weiter.

Weiter greifen bei einigen der bekannten Operationsgeräten sämtliche Sensoren und Aktoren, welche mechanischen Kontakt mit Elementen der Kassette benötigen, z.B. Druck- und Durchflusssensoren, direkt am Vakuumbehälter an. Für hohe und reproduzierbare Messgenauigkeiten und damit eine präzise elektronische Regelung des Fluidiksystems benötigen diese Sensoren stabile mechanische Schnittstellen. Der Vakuumbehälter muss daher in diesem Fall sehr steif ausgelegt sein, um Verformungen aufgrund des Vakuumaufbaus zu vermeiden, die sonst die Funktionsfähigkeit des mechatronischen und fluidischen Systems beeinträchtigen würden.

Kassettenmodule sind aus den Dokumenten US-A-5 499 969 und US-A-2008/114301 bekannt.

### DARSTELLUNG DER ERFINDUNG

Ausgehend von diesem Stand der Technik liegt der Erfindung eine Aufgabe zugrunde, ein Kassettenmodul oder Abflussbehälter, insbesondere für den Einsatz in der Augenchirurgie zur Aufnahme von bei einer Augenoperation aus dem Auge abgesogenem Material bzw. Flüssigkeiten, anzugeben, welches Kassettenmodul die Nachteile des Standes der Technik überwindet. Insbesondere soll das Kassettenmodul aber möglichst umweltgerecht entsorgbar und effizient herstellbar sein.

Eine solche Aufgabe löst der Gegenstand nach Anspruch 1. Demgemäss dient ein Kassettenmodul, insbesondere ein opthalmologisches Kassettenmodul, der Aufnahme von abgesogenen Flüssigkeiten oder Material. Das Kassettenmodul wird in einen Behälterschacht eines Absaugmoduls eingesetzt und dort unter Vakuum gesetzt. Das Kassettenmodul umfasst einen Behälter mit einer Behälterwand, welche einen Aufnahmeraum zur Aufnahme der Flüssigkeiten begrenzt, wobei der Behälter in den Behälterschacht einsetzbar ist. Der Behälter weist mindestens eine die Behälterwand durchdringende Einlassöffnung auf, durch welche Einlassöffnung die Flüssigkeit dem Aufnahmeraum des Behälters zuführbar ist. Weiter weist der Behälter mindestens eine die Behälterwand durchdringende Vakuumöffnung auf, über welche der Aufnahmeraum mit einem Unterdruck beaufschlagbar ist.

Dadurch, dass der Behälter über die mindestens eine Vakuumöffnung unter Unterdruck setzbar ist, kann ein Behälter geschaffen werden, welches sehr einfach ausgebildet ist. Hierdurch kann das Kassettenmodul kostengünstig und effizient hergestellt werden.

Die mindestens eine Vakuumöffnung ist vorzugsweise derart ausgebildet, dass diese einen einfachen Durchbruch in der Behälterwand darstellt. Unter einem einfachen Durchbruch wird eine Öffnung verstanden, welche sich von der einen Oberfläche der Behälterwand zur anderen Oberfläche der Behälterwand hindurch erstreckt, wobei keine flanschartigen oder ähnliche Elemente sich von der einen und der anderen Oberfläche weg erstrecken. Die Oberflächen der Seitenwand liegen typischerweise im Wesentlichen parallel zueinander. Herstellungsbedingt können die Oberflächen mit einem flachen Winkel im Bereich von ca. 1° bis max. 2° zueinander stehen. Auch hier kann von einer parallelen Ausbildung gesprochen werden.

Wenn sich der Behälter im Behälterschacht befindet, so liegt die mindestens eine Vakuumöffnung vorzugsweise beabstandet zu den Wänden des Behälterschachtes. Vakuum im Behälterschacht wirkt auch im Aufnahmeraum des Behälters über die Vakuumöffnung.

Das Kassettenmodul weist eine Dichtfläche auf, welche sich um die Behälterwand aussenseitig herum erstreckt und über welche eine Dichtwirkung zwischen Behälterschacht und dem in den Behälterschacht einragenden Behälter bereitstellbar ist. Aufgrund der Dichtwirkung wird bei eingesetztem Kassettenmodul der Aufnahmeraum und der Behälterschacht gegen die Umwelt hin abgeschlossen. Hierdurch kann also der Behälterschacht unter Unterdruck gesetzt werden, wobei der Unterdruck über die Vakuumöffnung auch im Aufnahmeraum entsprechend vorhanden ist.

Vorzugsweise verläuft die Dichtfläche in einer Ebene, welche sich vorzugsweise in einem Randbereich durch den Behälter hindurch erstreckt oder welche sich vorzugsweise durch eine Seitenwand des Behälters hindurch erstreckt.

Vorzugsweise ist auf der Dichtfläche eine um den Behälter herum verlaufende Dichtung angeordnet. Dies hat den Vorteil, dass keine Dichtung am Absaugsystem vorhanden sein muss und die Dichtung am Kasettenmodul ein Wegwerfelement ist, weshalb eine allfällige Wartung der Dichtung entfällt.

Alternativ ist die Dichtfläche mit einer am Absaugmodul angeordneten Dichtung in Kontakt bringbar ist. Die Dichtung ist in der Alternative also Teil des Absaugmoduls.

Vorzugsweise umfasst das Kassettenmodul mindestens einen Anschluss zur Verbindung an ein Operationssystem, welcher Anschluss in die Einlassöffnung mündet und eine Fluidleitung mit dem Anschluss in Verbindung steht. Über die Fluidleitung gelangt das Fluid über die Einlassöffnung in den Aufnahmeraum des Behälters.

Vorzugsweise umfasst das Kassettenmodul weiter ein Funktionsmodul, welches mit dem Behälter in Verbindung steht. Das Funktionsmodul dient der Aufnahme von diversen Elementen für verschiedene Funktionen.

Von der Dichtfläche her gesehen, erstreckt sich der Behälter vorzugsweise in eine erste Richtung und das Funktionsmodul in eine zweite Richtung, welche ungleich bzw. entgegengesetzt der ersten Richtung ist. Die Dichtfläche bildet also eine Art Trennfläche zwischen Funktionsmodul und Kassettenmodul.

Vorzugsweise ist die Behälterwand des Behälters aus Kunststoff und weist eine Wandstärke von 0.5 bis 1.5 mm, besonders bevorzugt von 0.7 bis 1.2 mm auf. Wandstärken in diesem Bereich gelten als instabil bei einer Wirkung von Vakuum auf die Innenseite des Behälters. Der Behälter weist unter gleichen Druckbedingungen innen und aussen eine vergleichsweise stabile Form auf, so dass dieser einfach zu handhaben ist. Ausserhalb des Behälters und im Aufnahmeraum herrscht im eingesetzten Zustand der gleiche Unterdruck.

Vorzugsweise wird der den Aufnahmeraum begrenzende Behälter durch eine umlaufende Seitenwand, eine mit der Seitenwand in Verbindung stehende Rückwand und einem gegenüber der Rückwand angeordneten Deckel bereitgestellt, wobei die Seitenwand und die Rückwand vorzugsweise einstückig ausgebildet sind und der Deckel vorzugsweise stoffschlüssig mit der Seitenwand verbunden wird. Seitenwand, Rückwand und Deckel stellen dabei die Behälterwand bereit, welche den Aufnahmeraum im Wesentlichen vollständig umgeben. Abgesehen von der Vakuumöffnung und der Einlassöffnung ist der Behälter fluiddicht ausgebildet.

In einer besonders bevorzugten Ausführungsform liegt, wenn der Behälter in einem Behälterschacht eines Absaugmoduls eingesetzt ist, die Behälterwand beabstandet zu den Wänden des Behälterschachtes. Dies insbesondere im Bereich der Vakuumöffnung.

In einer anderen Ausführungsform steht die Behälterwand mit den Wänden des Behälterschachtes in Kontakt. Im Bereich der Vakuumöffnung ist die Behälterwand aber dennoch vorzugsweise leicht beabstandet zu den Wänden des Behälterschachtes.

Vorzugsweise ist der Behälter aus Kunststoff und weist eine Eigenstabilität auf. Alternativ kann der aus Kunststoff gebildete Behälter auch die Gestalt einer Folie aufweisen. Die Ausbildung als Folie reduziert das Gewicht weiterhin. Die Dicke der Folie ist dabei kleiner als die oben genannte Wandstärke oder liegt im unteren Bereich der oben genannten Wandstärken.

Bezüglich der Zahl der Vakuumöffnungen verfügt der Behälter über mindestens eine oder mehrere Vakuumöffnungen. Die Vakuumöffnung erlaubt das Anlegen des Vakuums im Inneren des Behälters. Im eingesetzten Zustand liegen die Vakuumöffnung vorzugsweise im Bereich des Eintritts des Vakuumanschlusses in den Behälterschacht.

Vorzugsweise ist das Funktionsmodul am Behälter angeformt und das Funktionsmodul ist bevorzugt mit dem Deckel verschlossen.

Funktionsmodul und Behälter bilden im Wesentlichen eine einstückige und integrale Einheit aus Behälter und Funktionsmodul.

Vorzugsweise umfasst die Fluidleitung einen Schlauchabschnitt, der über eine konkave Rundung am Funktionsmodul geführt wird, wobei eine perilstaltische Pumpe mit dem Pumpenrad in die konkave Rundung einragt. Durch die perilstaltische Pumpe kann der Fluidfluss in der Fluidleitung unterstützt bzw. bereitgestellt werden. Dieser Schlauchabschnitt steht mit dem besagten Anschluss in Verbindung und mündet über die Einlassöffnung in den Behälter.

Vorzugsweise wird die Fluidleitung am Funktionsmodul abschnittsweise durch von einer Platte abstehende Stege und einer auf den Stegen liegenden und zur Platte befestigten Seitenwand geführt. In anderen Ausführungsformen kann auch auf die Platte verzichtet werden

Vorzugsweise verfügt das Kassettenmodul über eine Notentleerungsöffnung. Über die Notentleerungsöffnung kann die Flüssigkeit aus dem Behälter abgelassen werden, wenn dieser voll wird.

Ein Absaugsystem, insbesondere ein ophthalmologisches Absaugsystem, zur Aufnahme von bei einer Operation abgesogenem Material, wie Flüssigkeiten, umfasst ein Absaugmodul und ein Kassettenmodul nach der vorhergehenden Beschreibung. Das Absaugmodul umfasst einen durch Schachtwände begrenzten Behälterschacht zur Aufnahme des Behälters des Kassettenmoduls, welcher Behälterschacht über eine Einschuböffnung zugänglich ist, und eine Pumpe zur Erzeugung eines Unterdruckes im Behälterschacht. Der Unterdruck wirkt über die Vakuumöffnung auf den Behälter.

Die Einschuböffnung des Behälterschachtes ist durch Teile des Kassettenmoduls, insbesondere durch das Funktionsmodul und/oder den Deckel, luftdicht verschlossen. Das Kassettenmodul dient also nicht nur der Aufnahme der abgesogenen Flüssigkeit sondern auch dem luftdichten Abschluss des Behälterschachtes.

Vorzugsweise weist der Behälterschacht bezüglich der Aussenform des Behälters eine identische Form auf, so dass die Behälterwand des Behälters an der Schachtwand anliegt. Alternativ weist der Behälterschacht bezüglich der Aussenform des Behälters eine ähnliche Form auf, wobei die Behälterwand des Behälters beabstandet zur Schachtwand liegt.

Vorzugsweise liegt die Behälterwand im Bereich der Vakuumöffnungen beabstandet zur Schachtwand, so dass der Aufnahmeraum gut durch das im Behälterschacht bereitgestellte Vakuum beaufschlagt werden kann.

Vorzugsweise umfasst das Absaugmodul eine ebene Wand, welche eine um den Behälterschacht herum verlaufenden Dichtfläche zur Kontaktierung einer Dichtung im Bereich der Dichtfläche des Kassettenmoduls bereitstellt. Alternativ umfasst das Absaugmodul eine ebene Wand, auf welcher eine um den Behälterschacht herum verlaufende Dichtung zur Kontaktierung mit der Grundplatte angeordnet ist.

Die Dichtung kann auch in einer Rille angeordnet sein, welche sich in die Dichtfläche des Absaugmoduls oder des Kassettenmoduls hineinerstreckt.

Bevorzugt umfasst das Absaugmodul weiter einen in der Schachtwand angeordneten Unterdruckanschluss, wobei der Unterdruckanschluss bei eingesetztem Kassettenmodul im Bereich der mindestens einen Vakuumöffnung liegt, so dass der Unterdruckanschluss mit der Vakuumöffnung zusammenarbeiten kann. Der Unterdruckanschluss steht fluidisch, insbesondere pneumatisch, mit einer Pumpe in Verbindung.

Vorzugsweise ist das Kassettenmodul entlang einer geradlinig verlaufenden Einschubbewegung in das Absaugmodul einschiebbar.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Ansicht einer Ausführungsform eines Absaugsystems mit einem Absaugmodul und einem Kassettenmodul;
- Fig. 2: eine perspektivische Ansicht des Absaugmoduls nach Figur 1 ohne Kassettenmodul;
- Fig. 3: eine perspektivische Ansicht des Kassettenmoduls von Figur 1;
- Fig. 4: eine Explosionsansicht des Kassettenmoduls von Figur 1, wobei eine andere Variante des Deckels gezeigt wird;
- Fig. 5: eine weitere Perspektivansicht des Kassettenmoduls von Figur 1;
- Fig. 6: eine Rückansicht des Kassettenmoduls von Figur 1;
- Fig. 7: eine Schnittdarstellung entlang der Schnittlinie A-A gemäss Figur 1, wobei der Deckel nach der Variante von Figur 4 ausgebildet ist; und
- Fig. 8: eine Schnittdarstellung entlang der Schnittlinie B-B gemäss Figur 1, wobei der Deckel nach der Variante von Figur 4 ausgebildet ist.

### BESCHREIBUNG BEVORZUGTER AUSFÜHRUNGSFORMEN

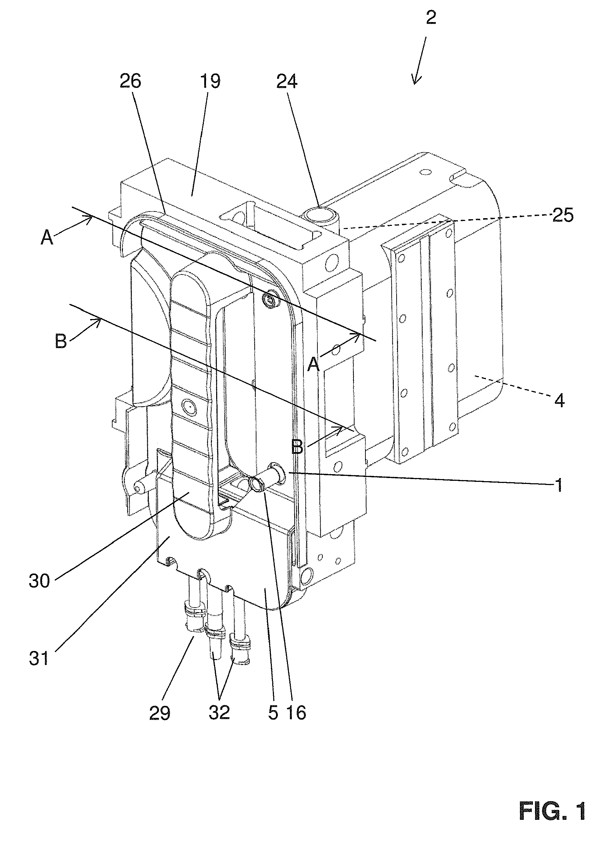

In der Fig. 1 wird ein Absaugsystem 2 mit einem Absaugmodul 19 und einem in das Absaugmodul 19 eingesetzten Kassettenmodul 1 gezeigt. Das Absaugsystem 2 ist vorzugsweise ein ophthalmologisches Absaugsystem. Das Absaugsystem 2 dient der Aufnahme von Flüssigkeiten und/oder Materialien, welche bei Operationen, insbesondere bei Augenoperationen, abgesogen werden. Vor einer Operation wird das Kassettenmodul 1 in das Absaugmodul 19 eingesetzt, während der Operation werden Teile des Kassettenmoduls 1 unter Unterdruck gesetzt und dienen der Aufnahme vom Material bzw. der Flüssigkeiten und nach der Operation wird das Kassettenmodul 1 wieder entfernt. Vorteilhafterweise ist das Kassettenmodul 1, wie nachfolgende Beschreibung zeigt, als Einwegmodul bereitgestellt, wodurch die Reinigung des Kassettenmoduls 1 entfällt. Das Kassettenmodul 1 wird also bevorzugt nur für eine einzige Operation eingesetzt.

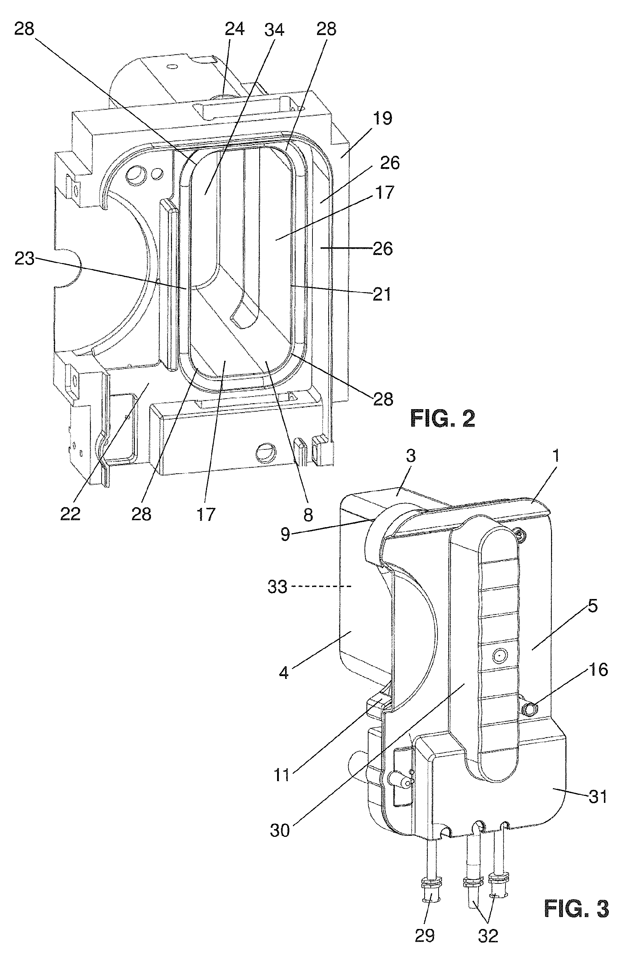

In der Fig. 2 wird das Absaugmodul 19 ohne das Kassettenmodul 1 gezeigt. Das Absaugmodul 19 umfasst im Wesentlichen einen durch Seitenwände 17 begrenzten Behälterschacht 8 zur Aufnahme des Kassettenmoduls 1, insbesondere zur Aufnahme des Behälters 4. Der Behälterschacht 8 ist über eine Einschuböffnung 21 zugänglich. Die Seitenwände 17 weisen hier abgerundete Ecken 28 auf. Der Behälterschacht 8 weist im Wesentlichen eine quaderförmige Gestalt auf. Gegenüber der Einschuböffnung 21 weist der Behälterschacht 8 eine Rückwand 34 auf. Bezüglich der Rückwand 34 kann auch gesagt werden, dass sich die Seitenwände 17 im Wesentlichen senkrecht von der Rückwand 34 erstrecken.

Von der Fig. 2 kann ebenfalls erkannt werden, dass das Absaugmodul 19 nebst dem Behälterschacht 8 noch ein weiterer optionaler Schacht 26 zur Aufnahme eines Funktionsmoduls 5, das ebenfalls Teil des Kassettenmoduls 1 sein kann, umfasst. Der optionale Schacht 26 liegt in Einschubrichtung gesehen vor dem Behälterschacht 8 und erstreckt sich hier seitlich über den Behälterschacht 8 hinaus.

In der Fig. 3 wird das Kassettenmodul 1 gezeigt, wobei das Kassettenmodul 1 in das Absaugmodul 19 gemäss den Fig. 1 und 2 einsetzbar ist. Das Kassettenmodul 1 umfasst einen Behälter 4, welcher durch eine Behälterwand 3 begrenzt ist. Die Behälterwand 3 begrenzt dabei einen Aufnahmeraum 33, welcher der Aufnahme des abgesaugten Materials bzw. der Flüssigkeit dient. Der Aufnahmeraum 33 wird in der gezeigten Ausführungsform durch eine umlaufende Seitenwand 38, eine mit der Seitenwand 38 in Verbindung stehende Rückwand 39 und einen gegenüber der Rückwand 39 angeordneten Deckel 37 begrenzt. Seitenwand 38 und Rückwand 39 sind vorzugsweise einstückig ausgebildet und der Deckel 37 wird vorzugsweise stoffschlüssig mit der Seitenwand 38 verbunden. Die Seitenwand 38, die Rückwand 39 und der Deckel 37 stellen die Behälterwand 3 bereit.

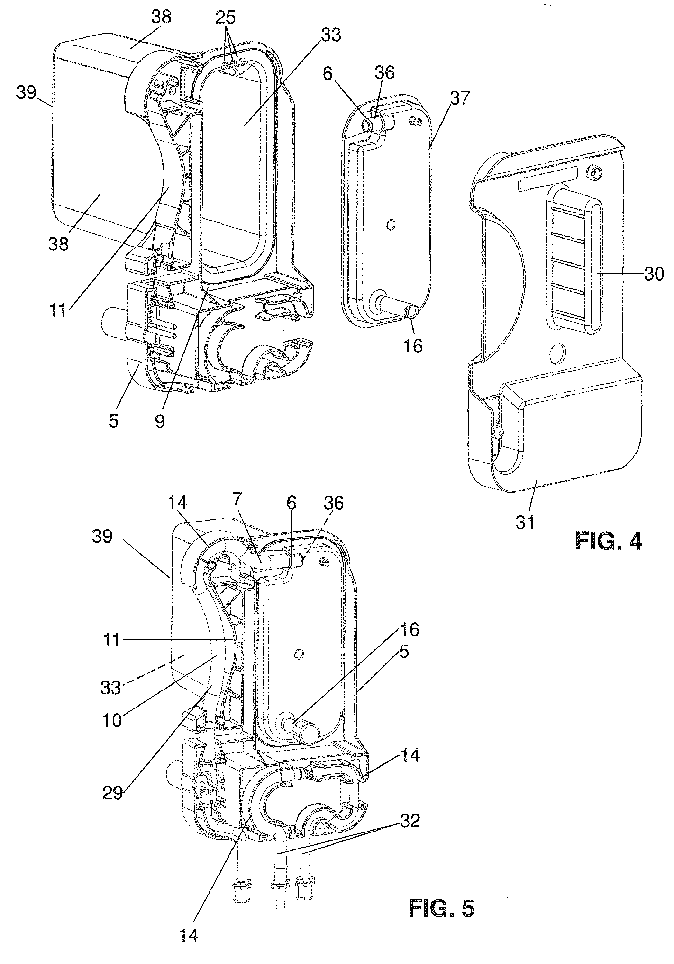

In der Figur 4 wird eine Explosionsdarstellung des Kassettenmoduls 1 gezeigt. Der Behälter 4 weist mindestens eine die Behälterwand 3 durchdringende Einlassöffnung 36 auf, durch welche Einlassöffnung 36 die Flüssigkeit dem Aufnahmeraum 33 des Behälters 4 zuführbar ist. Die Einlassöffnung 36 ist hier im Deckel 37 angeordnet. In anderen Ausführungsformen kann die Einlassöffnung 36 an beliebiger Stelle in der Behälterwand 3 liegen. Weiter weist der Behälter 4 mindestens eine die Behälterwand 3 durchdringende Vakuumöffnung 25 auf, über welche der Aufnahmeraum 33 mit einem Unterdruck beaufschlagbar ist.

Das Kassettenmodul 1 weist mindestens einen Anschluss 6 zur Verbindung an ein Operationssystem und mindestens eine sich dem Anschluss 6 anschliessende und in den Behälter mündende Fluidleitung 7 auf. Der Anschluss 6 weist die Einlassöffnung 36 auf. Die Fluidleitung 7 mündet über eine Einlassöffnung 36 in den Aufnahmeraum 33. Die Fluidleitung 7 lässt sich in der Fig. 5 besonders gut erkennen. Die Fluidleitung 7 ist vorzugsweise ein Schlauch 29.

Weiter umfasst das Kassettenmodul 1 ein Funktionsmodul 5, welches mit dem Behälter 4 in Verbindung steht. In den Figuren 3 bis 5 lässt sich das Funktionsmodul 5 gut erkennen. Weiter sind im Funktionsmodul 5 Zusatzschläuche 32 untergebracht. Die Schläuche 32 haben unterschiedlichste Funktionen, dienen aber im Wesentlichen der Unterstützung des Absaugvorgangs. Die Zusatzschläuche 32 dienen als Zuleitungselemente, beispielsweise für Infusionsflüssigkeiten.

Der Behälter 4 bzw. die Behälterwand 3 können mit einer vergleichsweise dünnen Wandstärke ausgestattet sein. Da die Behälterwand 3 im eingesetzten Zustand von aussen und von innen her mit einem Unterdruck beaufschlagt wird, wirken im Absaugbetrieb keine Kräfte auf den Behälter. Insofern kann der Behälter 4 auch derart ausgebildet sein, dass dieser Nicht-Vakuumfest ist.

Der Behälter 4 ist bevorzugt in einer Variante derart ausgebildet, dass dieser in Kontakt mit den Seitenwänden 17 des Absaugmoduls 19 kommt, wobei die Behälterwände 3 an den Seitenwänden 17 abgestützt sind. In einer anderen Variante ist diese Abstützung nicht nötig und der Behälter 4 kann beabstandet zu den Wänden 17 liegen. Diese Variante ist vorteilhaft, weil das Anlegen des Vakuums einfacher zu handhaben ist.

Die Behälterwand 3 ist vorzugsweise aus einem Kunststoff und weist eine Wandstärke von 0.5 bis 1.5 mm, besonders bevorzugt von 0.7 bis 1.2 mm auf. Solche Wände sind typischerweise bei Einwirkung eines Unterdruckes im Aufnahmeraum 33 als instabil zu betrachten. In einer alternativen Ausführungsform kann der Behälter 4 auch aus Kunststoff in der Gestalt einer Folie sein.

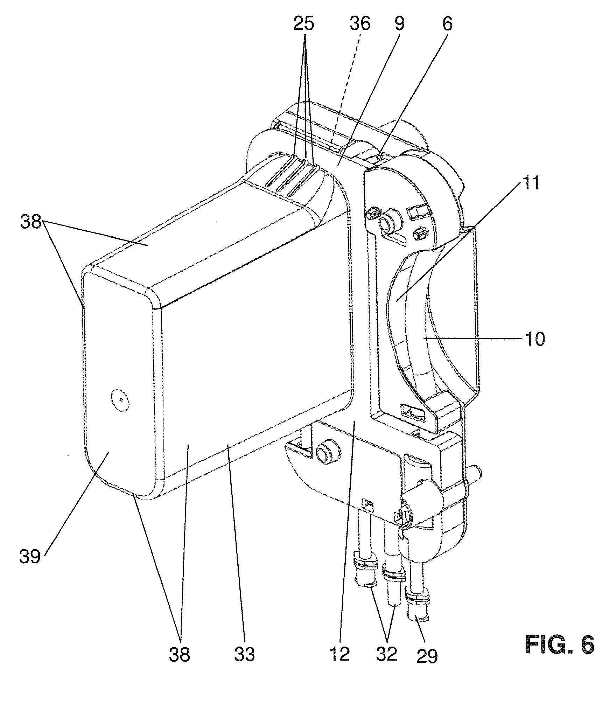

Von der Fig. 6 kann die mindestens eine Vakuumöffnung 25 gut erkannt werden. In dieser Ausführungsform sind mehrere Schlitze als Vakuumöffnungen 25 parallel zueinander angeordnet. In anderen Ausführungsformen können auch mehrere Vakuumöffnungen 25 in einem Raster angeordnet sein. Man kann auch von einem Set Vakuumöffnungen 25 sprechen. Über diese Öffnungen 25 lässt sich ein Vakuum im Inneren des Behälters 4 anlegen.

Der Behälter 4 und das Funktionsmodul 5 sind an einer gemeinsamen Grundplatte 9 angeordnet. Die Grundplatte 9 hat dabei im Wesentlichen die Funktion als Basiselement für den Behälter 4 und das Funktionsmodul 5. Die Fluidleitung 7 bzw. die Schläuche 29 und 32, verlaufen teilweise durch die Grundplatte 9.

Der Behälter 4, das Funktionsmodul 5 und die Grundplatte 9 stellen eine integrale einstückige Einheit bereit. Besonders bevorzugt werden Teile dieser Einheit mit Spritzgussverfahren hergestellt. Diese Teile sind insbesondere die Seitenwand 38 und die Rückwand 39, welche einstückig sind. Der Deckel 37 kann ebenfalls mit einem Spritzgussverfahren hergestellt werden, wobei der Behälter 4 mit einem Schweiss- und/oder Klebeverfahren komplettiert wird, indem der Deckel 37 der Einheit aus Seitenwand 38 und Rückwand 39 angeschweisst wird. In diesem Zusammenhang wird auf die Figur 4 verwiesen. Nach vorne gegen das Funktionsmodul 5 hin, wird der Aufnahmeraum 33 mit einem Deckel 37 verschlossen. Der Deckel 37 wird über einen Schweiss- und/oder Klebeverfahren mit dem Behälter 4 verbunden. Besonders bevorzugt wird ein Ultraschallschweissverfahren eingesetzt.

Von der Fig. 3 kann weiter erkannt werden, dass am Kassettenmodul 1 ein Handgriff 30 angeformt ist. Mit dem Handgriff 30 kann der Operateur das Kassettenmodul 1 einfach ergreifen und in den Behälterschacht 8 des Absaugmoduls 19 einschieben beziehungsweise entfernen.

Die Fluidleitung 7, welche in den Innenraum 33 des Behälters 4 mündet, umfasst einen Schlauchabschnitt 10, der über eine konkave Rundung 11 am Funktionsmodul geführt wird, was in den Figuren 4 und 5 besonders gut gezeigt wird. Die konkave Rundung 11 dient im Wesentlichen der Aufnahme eines hier nicht gezeigten Pumpenrades einer peristaltischen Pumpe. Das Pumpenrad ragt in die konkave Rundung 11 ein, wobei sich das Pumpenrad zum feststehenden Schlauchabschnitt 10 dreht. Die Drehbewegung erzeugt eine konstante Förderung des sich im Schlauchabschnitt 10 befindlichen Mediums.

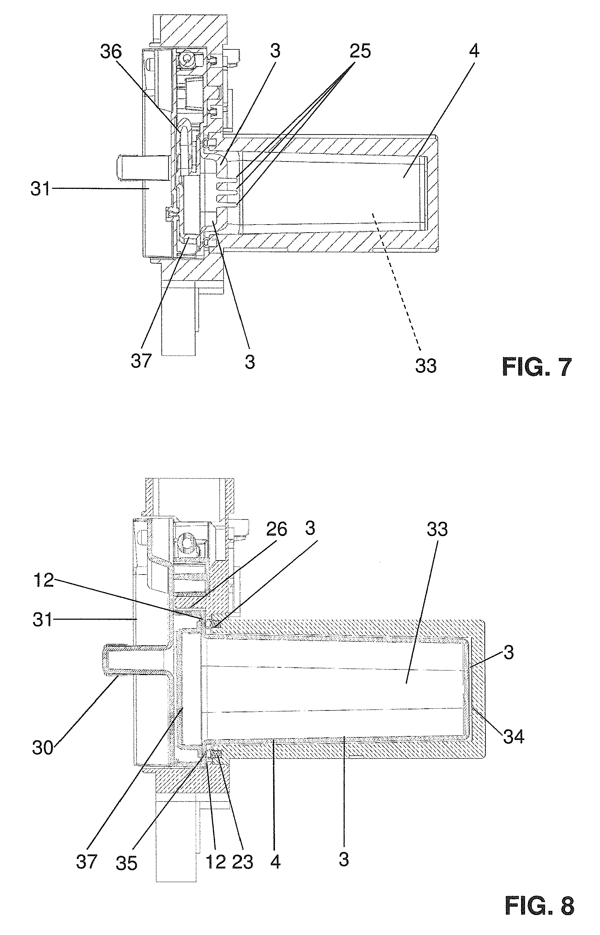

Die Fig. 7 zeigt einen Schnitt entlang der Schnittlinie A-A der Figur 1 und die Fig. 8 zeigt einen Schnitt in der gleichen Ebene, welcher aber auf der Höhe der Schnittlinie B-B liegt.

In den Figuren 7 und 8 kann gut erkannt werden, dass die Behälterwände 3 den besagten Behälterinnenraum 33 bereitstellen. Auch wird gezeigt, dass nach vorne hin der Behälterinnenraum 33 durch den Deckel 37 verschlossen wird. Der Deckel 37 wird beispielsweise über eine Ultraschallschweissung 35 mit dem Behälter 4 verbunden.

Der Behälter 4 umfasst weiter eine Dichtfläche 12, welche hier durch eine Grundplatte 9 bereitgestellt wird. Diese Dichtfläche 12 wird ebenfalls in der Fig. 8 gut gezeigt. Die Dichtfläche 12 arbeitet mit einem Dichtungselement 23, das am Absaugmodul 19 angeordnet ist, zusammen. Folglich wird zwischen dem Dichtungselement 23 und der Dichtfläche 12 eine Dichtwirkung bereitgestellt. Alternativ kann auf der Dichtfläche 12 auch eine Dichtung angeordnet sein, welche dann mit einer entsprechenden Dichtfläche am Absaugmodul 19 zusammen arbeitet. Die Dichtung kann also am Kassettenmodul 1 und/oder am Absaugmodul 19 angeordnet werden.

Die Fluidleitungen 7, 29 und 32 können, wie dies in den Fig. 4 und 5 gezeigt wird, durch von einer Platte 9 abstehende Stege 14 am Funktionsmodul 5 geführt werden. Die Stege 14 können beispielsweise noch mit einem Deckel 31 abgedeckt werden.

Das Kassettenmodul 1 verfügt weiter über eine Notentleerungsöffnung 16. Die Notentleerungsöffnung 16 ist hier als Durchbruch durch den Deckel 37 bereitgestellt. Die Notentleerungsöffnung 16 stellt dabei einen Durchgang in den Behälterinnenraum 33 des Behälters 4 bereit. Sollte der Behälter 4 während einer Operation zu einem grossen Mass gefüllt werden, so kann das Operationspersonal in einer Pause über einen Schlauch die Flüssigkeit aus dem Innenraum 33 des Behälters 4 entfernen. Für die Dauer der Notentleerung wird die Beaufschlagung des Behälterinnenraums 33 mit dem Vakuum gestoppt.

Die Fig. 7 und 8 zeigen, dass der Behälterschacht 8 bezüglich der Aussenform des Behälters 4 eine identische Gestalt bzw. eine komplementäre oder passende Form aufweist. Somit liegt also die Behälterwand 3 des Behälters 4 an der Schachtwand 17 des Behälterschachtes 8 an. Vorzugsweise gibt es ein kleines Spiel zwischen der Behälterwand 3 und dem Behälterschacht 8.

Von der Fig. 2 kann weiter gut erkannt werden, dass hier auch das Absaugmodul 19 eine ebene Wand 22 umfasst, welche um den Behälterschacht 8 herum verläuft. Die Dichtung 23 kann in der ebenen Wand 22 eingelassen sein oder auf dieser aufliegen. Die Dichtung 23 kann dabei, wie in der Figur 2 gezeigt am Absaugmodul 19 angeordnet sein oder alternativ an der Dichtfläche 23 des Kassettenmoduls 1.

Das Absaugmodul 2 umfasst weiter einen in der Schachtwand 17 des Behälterschachtes 8 angeordneten Unterdruckanschluss 24, welcher mit der Pumpe in Verbindung steht. Dieser Unterdruckanschluss 24 wird in der Fig. 1 gezeigt. Der Unterdruckanschluss 24 ist dabei so angeordnet, dass dieser mit der mindestens einen Vakuumöffnung 25 in der Behälterwand 3 des Behälters 4 zusammenarbeitet. Vorzugsweise so, dass die mindestens eine Vakuumöffnung 25 im Behälter 4 in den Wirkbereich des Unterdruckanschlusses 24 zu liegen kommt.

Von den Fig. 1 bis 3 kann auch gut erkannt werden, dass das Kassettenmodul 1 entlang einer gradlinig verlaufenden Einschubbewegung in das Absaugmodul 19 einschiebbar ist. Bei der Entnahme des Kassettenmoduls 1 aus dem Absaugmodul 19 bzw. dem Behälterschacht 8 des Absaugmoduls 19 kann das Kassettenmodul 1 wieder in entgegengesetzte Richtung entfernt werden.

Neben dem Behälterschacht 8 umfasst das Absaugmodul 19 noch einen zusätzlichen Schacht 26, in welchem das Funktionsmodul 5 einschiebbar ist. Dieser Schacht 26 liegt dabei vor dem Behälterschacht 8.

Zusammenfassend weist das erfindungsgemässe Kassettenmodul 1 den Vorteil auf, dass dieses sehr einfach ausgebildet und über die Vakuumöffnung mit einem Unterdruck im Berhälterschacht beaufschlagbar ist.

### BEZUGSZEICHENLISTE

- 1: Kassettenmodul
- 2: Absaugvorrichtung
- 3: Behälterwand
- 4: Behälter
- 5: Funktionsmodul
- 6: Anschluss
- 7: Fluidleitung
- 8: Behälterschacht
- 9: Grundplatte
- 10: Schlauchabschnitt
- 11: Rundung
- 12: Dichtfläche
- 14: Stege
- 16: Notentleerungsöffnung
- 17: Seitenwände
- 19: Absaugmodul
- 21: Einschuböffnung
- 22: ebene Wand
- 23: Dichtung
- 24: Unterdruckanschluss
- 25: Vakuumöffnung
- 26: optionaler Schacht
- 28: abgerundete Ecken
- 29: Schläuche
- 30: Handgriff
- 31: Deckel
- 32: Zusatzschläuche
- 33: Aufnahmeraum
- 34: Rückwand
- 35: Ultraschallschweissung
- 36: Einlassöffnung
- 37: Deckel
- 38: Seitenwände
- 39: Rückwand

## Patentansprüche

1. Kassettenmodul (1), insbesondere ein ophthalmologisches Kassettenmodul (1), zur Aufnahme von bei einer Operation abgesogenem Material und/oder Flüssigkeiten, und zum Einsetzen in einen Behälterschacht (8) eines Absaugmoduls (19) umfassend
einen Behälter (4) mit einer Behälterwand (3), welche einen Aufnahmeraum (33) zur Aufnahme der Flüssigkeiten begrenzt, wobei der Behälter (4) in den Behälterschacht (8) einsetzbar ist, wobei
der Behälter (4) mindestens eine die Behälterwand (3) durchdringende Einlassöffnung (36) aufweist, durch welche Einlassöffnung (36) das Material und/oder die Flüssigkeit dem Aufnahmeraum (33) des Behälters (4) zuführbar ist, und wobei
der Behälter (4) mindestens eine die Behälterwand (3) durchdringende Vakuumöffnung (25) aufweist, über welche der Aufnahmeraum (33) mit einem im Behälterschacht (8) aufgebauten Unterdruck beaufschlagbar ist,
**dadurch gekennzeichnet, dass** das Kassettenmodul (1) weiter eine Dichtfläche (12) aufweist, welche sich um die Behälterwand (3) aussenseitig herum erstreckt und über welche eine Dichtwirkung zwischen Behälterschacht (8) und dem in den Behälterschacht (8) einragenden Behälter (4) bereitstellbar ist.

2. Kassettenmodul (1) nach Anspruch 1, **dadurch gekennzeichnet, dass** die Dichtfläche (12) in einer Ebene verläuft, welche sich vorzugsweise in einem Randbereich durch den Behälter (4) hindurch erstreckt, oder welche sich vorzugsweise durch eine Seitenwand des Behälters (4) hindurch erstreckt.

3. Kassettenmodul (1) nach Anspruch 1 oder 2, **dadurch gekennzeichnet, dass** auf der Dichtfläche (12) eine um den Behälter (4) herum verlaufende Dichtung (23) angeordnet ist oder dass die Dichtfläche (12) mit einer am Absaugmodul (19) angeordneten Dichtung in Kontakt bringbar ist.

4. Kassettenmodul (1) nach einem der vorhergehenden Ansprüche, **dadurch gekennzeichnet, dass** das Kassettenmodul (1) mindestens einen Anschluss (6) zur Verbindung an ein Operationssystem umfasst, welcher Anschluss (6) in die Einlassöffnung (36) mündet und eine Fluidleitung (7), insbesondere ein Schlauchabschnitt (29), mit dem Anschluss (6) in Verbindung steht.

5. Kassettenmodul (1) nach einem der vorhergehenden Ansprüche, **dadurch gekennzeichnet, dass** das Kassettenmodul (1) weiter ein Funktionsmodul (5) umfasst, welches mit dem Behälter (4) in Verbindung steht, wobei von der Dichtfläche (12) her gesehen, sich der Behälter (4) vorzugsweise in eine erste Richtung und das Funktionsmodul (5) vorzugsweise in eine zweite Richtung, welche ungleich bzw. entgegengesetzt zur der ersten Richtung ist, erstreckt.

6. Kassettenmodul (1) nach einem der vorhergehenden Ansprüche, **dadurch gekennzeichnet, dass** die Behälterwand (3) mit einer vergleichsweise dünnen Wandstärke ausgestattet ist, wobei die Wandstärke vorzugsweise im Bereich von 0.5 bis 1.5 mm, besonders bevorzugt von 0.7 bis 1.2 mm, liegt.

7. Kassettenmodul (1) nach einem der vorhergehenden Ansprüche, **dadurch gekennzeichnet, dass** wenn der Behälter (4) in einem Behälterschacht (8) eines Absaugmoduls (19) eingesetzt ist, die Behälterwand (3) beabstandet zu den Wänden des Behälterschachtes (8) liegt oder dass die Behälterwand (3) durch den Behälterschacht (8) gestützt wird.

8. Kassettenmodul (1) nach einem der vorhergehenden Ansprüche, **dadurch gekennzeichnet, dass** der Behälter (4) aus Kunststoff ist und eine Eigenstabilität aufweist, oder dass der Behälter (4) aus Kunststoff ist und die Gestalt einer Folie aufweist.

9. Kassettenmodul (1) nach einem der vorhergehenden Ansprüche, **dadurch gekennzeichnet, dass** mehrere Vakuumöffnungen (25) benachbart zueinander angeordnet sind, wobei die Vakuumöffnungen (25) bevorzugt die Form von Schlitzen oder kreisrunden Öffnungen aufweisen.

10. Kassettenmodul (1) nach einem der vorhergehenden Ansprüche, **dadurch gekennzeichnet, dass** der den Aufnahmeraum (33) begrenzende Behälter (4) durch eine umlaufende Seitenwand (38), eine mit der Seitenwand (38) in Verbindung stehende Rückwand (39) und einem gegenüber der Rückwand (39) angeordneten Deckel (37) bereitgestellt wird, wobei die Seitenwand (38) und die Rückwand (39) vorzugsweise einstückig ausgebildet sind und der Deckel (37) vorzugsweise stoffschlüssig mit der Seitenwand (39) verbunden wird.

11. Kassettenmodul (1) nach einem der vorhergehenden Ansprüche, **dadurch gekennzeichnet,**
**dass** die Fluidleitung (7) einen Schlauchabschnitt (10) umfasst, der über eine konkave Rundung (11) am Funktionsmodul (5) geführt wird, welche Rundung der Aufnahme eines Pumpenrades einer perilstaltische Pumpe dient,
und/oder dass die Fluidleitung (7) am Funktionsmodul (5) abschnittsweise durch von einer Platte (9) abstehende Stege (14) und/oder einer auf den Stegen (14) liegenden und zur Platte (9) befestigten Seitenwand geführt,
und/oder dass das Kassettenmodul (1) über eine Notentleerungsöffnung (16) verfügt
und/oder dass das Funktionsmodul (5) am Behälter (4) angeformt ist, wobei das Funktionsmodul (5) mit einem Deckel (37) verschlossen ist.

12. Absaugsystem (2), insbesondere ein ophthalmologisches Absaugsystem (2), zur Aufnahme von bei einer Operation abgesogenem Material, wie Flüssigkeiten, umfassend ein Absaugmodul (19) und ein Kassettenmodul (1) nach einem der vorhergehenden Ansprüche,
wobei das Absaugmodul (19) einen durch Schachtwände (17) begrenzten Behälterschacht (8) zur Aufnahme des Behälters (4) des Kasettenmoduls (1), welcher Behälterschacht (8) über eine Einschuböffnung (21) zugänglich ist, und eine Pumpe zur Erzeugung eines Unterdruckes im Behälterschacht (8) umfasst.

13. Absaugsystem (2) nach Anspruch 12, **dadurch gekennzeichnet, dass** die Einschuböffnung (21) des Behälterschachtes (8) durch Teile des Kassettenmoduls (1), insbesondere durch das Funktionsmodul und/oder den Deckel, luftdicht verschlossen ist.

14. Absaugsystem (2) nach Anspruch 12 oder 13, **dadurch gekennzeichnet, dass** der Behälterschacht (8) bezüglich der Aussenform des Behälters (4) eine identische Form aufweist, so dass die Behälterwand (3) des Behälters (4) an der Schachtwand (17) anliegt, oder dass er Behälterschacht (8) bezüglich der Aussenform des Behälters (4) eine ähnliche Form aufweist, wobei die Behälterwand (3) des Behälters (4) beabstandet zur Schachtwand (17) liegt.

15. Absaugsystem (2) nach einem der Ansprüche 12 bis 14, **dadurch gekennzeichnet, dass** das Absaugmodul (19) eine ebene Wand (22) umfasst, welche eine um den Behälterschacht (8) herum verlaufende Dichtfläche zur Kontaktierung mit einer im Bereich Dichtfläche (9) des Kassettenmoduls angeordneten Dichtung bereitstellt.

16. Absaugsystem (2) nach einem der Ansprüche 12 bis 15, **dadurch gekennzeichnet, dass** das Absaugmodul (19) weiter mindestens einen in der Schachtwand (17) angeordneten Unterdruckanschluss (24) umfasst, wobei der Unterdruckanschluss (24) bei eingesetztem Kassettenmodul (1) vorzugsweise im Bereich der mindestens einen Vakuumöffnung (25) liegt, und wobei der Unterdruckanschluss mit der Pumpe fluidisch in Verbindung steht.

17. Absaugsystem (2) nach einem der Ansprüche 14 bis 16, **dadurch gekennzeichnet, dass** das Kassettenmodul (1) entlang einer geradlinig verlaufenden Einschubbewegung in das Absaugmodul (19) einschiebbar ist.

## Claims

1. Cassette module (1), in particular an ophthalmological cassette module (1), for receiving material and/or liquids aspirated during an operation and for insertion into a container chamber (8) of a suction module (19), comprising
a container (4) with a container wall (3) that delimits a receiving space (33) for receiving the liquids, wherein the container (4) can be inserted into the container chamber (8), wherein
the container (4) has at least one inlet opening (36) extending through the container wall (3), through which inlet opening (36) the material and/or the liquid can be delivered to the receiving space (33) of the container (4), and wherein
the container (4) has at least one vacuum opening (25) which extends through the container wall (3) and by way of which the receiving space (33) can be subjected to an underpressure built up in the container chamber (8),
**characterized in that** the cassette module (1) moreover has a sealing surface (12) which extends around the outside of the container wall (3) and by which a sealing action can be provided between the container chamber (8) and the container (4) protruding into the container chamber (8).

2. Cassette module (1) according to Claim 1, **characterized in that** the sealing surface (12) runs in a plane which preferably extends in an edge area through the container (4) or which preferably extends through a side wall of the container (4).

3. Cassette module (1) according to Claim 1 or 2, **characterized in that** a seal (23) extending around the container (4) is arranged on the sealing surface (12), or **in that** the sealing surface (12) can be brought into contact with a seal arranged on the suction module (19).

4. Cassette module (1) according to one of the preceding claims, **characterized in that** the cassette module (1) comprises at least one port (6) for connection to an operating system, which port (6) opens into the inlet opening (36) and connects a fluid line (7), in particular a hose section (29), to the port (6).

5. Cassette module (1) according to one of the preceding claims, **characterized in that** the cassette module (1) moreover comprises a function module (5), which is in connection with the container (4), wherein, seen from the direction of the sealing surface (12), the container (4) preferably extends in a first direction and the function module (5) preferably extends in a second direction that is different from or counter to the first direction.

6. Cassette module (1) according to one of the preceding claims, **characterized in that** the container wall (3) is provided with a comparatively thin wall thickness, wherein the wall thickness preferably lies in the range of 0.5 to 1.5 mm, particularly preferably of 0.7 to 1.2 mm.

7. Cassette module (1) according to one of the preceding claims, **characterized in that**, when the container (4) is inserted in a container chamber (8) of a suction module (19), the container wall (3) lies at a distance from the walls of the container chamber (8), or **in that** the container wall (3) is supported by the container chamber (8).

8. Cassette module (1) according to one of the preceding claims, **characterized in that** the container (4) is made of plastic and has an inherent stability, or **in that** the container (4) is made of plastic and has the form of a film.

9. Cassette module (1) according to one of the preceding claims, **characterized in that** several vacuum openings (25) are arranged adjacent to one another, wherein the vacuum openings (25) preferably have the form of slits or circular openings.

10. Cassette module (1) according to one of the preceding claims, **characterized in that** the container (4) delimiting the receiving space (33) is provided by a circumferential side wall (38), a rear wall (39) connected to the side wall (38), and a lid (37) arranged opposite the rear wall (39), wherein the side wall (38) and the rear wall (39) are preferably formed in one piece, and the lid (37) is preferably cohesively bonded to the side wall (39).

11. Cassette module (1) according to one of the preceding claims, **characterized in that**
the fluid line (7) comprises a hose section (10) that is guided over a concave rounding (11) on the function module (5), which rounding serves to receive a pump wheel of a peristaltic pump,
and/or **in that** the fluid line (7) is guided on the function module (5) partly by webs (14) protruding from a plate (9) and/or by a side wall lying on the webs (14) and secured to the plate (9),
and/or **in that** the cassette module (1) has an emergency drainage opening (16), and/or **in that** the function module (5) is integrally formed on the container (4), wherein the function module (5) is closed with a lid (37).

12. Suction system (2), in particular an ophthalmological suction system (2), for receiving material, such as liquids, aspirated during an operation, comprising a suction module (19) and a cassette module (1) according to one of the preceding claims,
wherein the suction module (19) comprises a container chamber (8) which is delimited by chamber walls (17) and which is used for receiving the container (4) of the cassette module (1), which container chamber (8) is accessible via an insert opening (21), and a pump for generating an underpressure in the container chamber (8).

13. Suction system (2) according to Claim 12, **characterized in that** the insert opening (21) of the container chamber (8) is closed in an airtight manner by parts of the cassette module (1), in particular by the function module and/or the lid.

14. Suction system (2) according to Claim 12 or 13, **characterized in that** the container chamber (8) has a shape identical to the outer shape of the container (4), such that the container wall (3) of the container (4) bears on the chamber wall (17), or **in that** the container chamber (8) has a shape similar to the outer shape of the container (4), in which case the container wall (3) of the container (4) lies at a distance from the chamber wall (17).

15. Suction system (2) according to one of Claims 12 to 14, **characterized in that** the suction module (19) comprises a plane wall (22), which provides a sealing surface extending around the container chamber (8) for contact with a seal arranged in the area of the sealing surface (9) of the cassette module.

16. Suction system (2) according to one of Claims 12 to 15, **characterized in that** the suction module (19) moreover comprises at least one underpressure port (24) arranged in the chamber wall (17), wherein the underpressure port (24) lies preferably in the area of the at least one vacuum opening (25) when the cassette module (1) is inserted, and wherein the underpressure port is connected fluidically to the pump.

17. Suction system (2) according to one of Claims 14 to 16, **characterized in that** the cassette module (1) can be pushed into the suction module (19) in a rectilinearly oriented insertion movement.

## Revendications

1. Module de cassette (1), en particulier un module de cassette ophtalmologique (1), pour la réception de matériel et / ou de liquides aspirés lors d'une opération et pour l'insertion dans un puits de réception (8) d'un module d'aspiration (19), comprenant
un récipient (4) ayant une paroi de récipient (3) qui limite un espace de réception (33) pour la réception des fluides, le conteneur (4) pouvant être inséré dans le puits de réception (8),
dans lequel le récipient (4) présente au moins une ouverture d'entrée (36) traversant la paroi de récipient (3), à travers de laquelle ouverture d'entrée (36) le matériel et / ou le liquide peut être amené dans l'espace de réception (33) du récipient (4), et
dans lequel le récipient (4) présente au moins une ouverture de vide (25) traversant la paroi de récipient (3), à travers de laquelle une sous-pression établie dans le puits de réception (8) peut être appliquée à l'espace de réception (33),
**caractérisé en ce que** le module de cassette (1) comprend d'avantage une surface d'étanchéité (12) s'étendant autour du côté extérieur de la paroi de récipient (3) et à travers de laquelle un effet étanchéifiant entre le puits de réception (8) et le récipient (4) pénétrant dans le puits de réception (8) peut être pourvu.

2. Module de cassette (1) selon la revendication 1, **caractérisé en ce que** la surface d'étanchéité (12) s'étend dans un plan, lequel s'étend préférablement, dans un domaine périphérique, à travers le récipient (4), ou lequel s'étend préférablement à travers une paroi latérale du récipient (4).

3. Module de cassette (1) selon la revendication 1 ou 2, **caractérisé en ce que** un joint (23) est disposé sur la surface d'étanchéité (12), s'étendant autour du récipient (4) ou **en ce que** la surface d'étanchéité (12) peut être mise en contact avec un joint disposé au niveau du module d'aspiration (19).

4. Module de cassette (1) selon une des revendications précédentes, **caractérisé en ce que** le module de cassette (1) comprend au moins un raccordement (6) pour la connexion à un système d'opération, lequel raccord (6) débouche dans l'ouverture d'entrée (36) et une conduite de fluide (7), en particulier une section de tuyau (29), est en connexion avec le raccord (6).

5. Module de cassette (1) selon une des revendications précédentes, **caractérisé en ce que** le module de cassette (1) comprend d'avantage un module de fonction (5), lequel est en connexion avec le récipient (4), dans lequel vu depuis la surface d'étanchéité (12), le récipient (4) s'étend préférablement dans une première direction et le module de fonction (5) s'étend préférablement dans une deuxième direction, laquelle est non identique respectivement opposée à la première direction.

6. Module de cassette (1) selon une des revendications précédentes, **caractérisé en ce que** la paroi de récipient (3) est pourvue d'une épaisseur de paroi comparativement faible, où l'épaisseur de paroi est préférablement située dans le domaine de 0.5 à 1.5 mm, en particulier préférablement de 0.7 à 1.2 mm.

7. Module de cassette (1) selon une des revendications précédentes, **caractérisé en ce que** le récipient (4) est inséré dans un puits de réception (8) d'un module d'aspiration (19), la paroi de récipient (3) est disposée de manière espacée par rapport aux parois du puits de réception (8) ou que la paroi de récipient (3) est soutenue par le puits de réception (8).

8. Module de cassette (1) selon une des revendications précédentes, **caractérisé en ce que** le récipient (4) est fait de plastique et présente une stabilité propre, ou que le récipient (4) est fait en plastique et présente une forme de film.

9. Module de cassette (1) selon une des revendications précédentes, **caractérisé en ce que** plusieurs ouvertures de vide (25) sont disposées l'une avoisinant l'autre, où les ouvertures de vide (25) présentent préférablement une forme de fente ou d'ouvertures circulaires.

10. Module de cassette (1) selon une des revendications précédentes, **caractérisé en ce que** le récipient (4) limitant l'espace de réception (33) est pourvu par une paroi latérale suivant la périphérie (38), une paroi postérieure (39) étant en connexion avec la paroi latérale (38) et un couvercle (37) étant disposé face à la paroi postérieure (39), où la paroi latérale (38) et la paroi postérieure (39) sont préférablement formées en une pièce et le couvercle (37) est préférablement connecté par liaison de matière avec la paroi latérale (39).

11. Module de cassette (1) selon une des revendications précédentes, **caractérisé en ce que**
la conduite de fluide (7) comprend une section de tuyau (10), laquelle est guidée à travers un arrondi concave (11) au niveau du module de fonction (5), lequel arrondi sert à la réception d'une roue de pompe d'une pompe péristaltique,
et/ou que la conduite de fluide (7) est guidée au niveau du module de fonction (5) sur des sections par des âmes (14) saillants d'une plaque (9) et/ou par une paroi latérale reposant sur les âmes (14) et fixée à la plaque (9),
et/ou que le module de cassette (1) dispose d'une ouverture d'évacuation d'urgence (16)
et/ou que le module de fonction (5) est formée sur un récipient (4), où le module de fonction (5) est fermé au moyen d'un couvercle (37).

12. Système d'aspiration (2), en particulier un système d'aspiration ophtalmologique (2), pour la réception de matériel aspiré lors d'une opération, tel que des fluides, comprenant un module d'aspiration (19) et un module de cassette (1) selon une des revendications précédentes, où le module d'aspiration (19) comprend un puits de réception (8) limité par des parois de puits (17) pour la réception d'un récipient (4) du module de cassette (1), lequel puits de réception (8) est accessible à travers une ouverture d'insertion (21) et une pompe pour la création d'une sous-pression dans le puits de réception (8).

13. Système d'aspiration (2) selon la revendication 12, **caractérisé en ce que** l'ouverture d'insertion (21) du puits de réception (8) est fermée de manière étanche à l'air par des parties du module de cassette (1), en particulier par le module de fonction et/ou le couvercle.

14. Système d'aspiration (2) selon la revendication 12 ou 13, **caractérisé en ce que** le puits de réception (8) présente par rapport à la forme extérieure du récipient (4) une forme identique, de sorte que la paroi de récipient (3) du récipient (4) est adjacente à la paroi du puits (17), ou que le puits de réception (8) présente par rapport à la forme extérieure du récipient une forme similaire, où la paroi de récipient (3) du récipient (4) est disposé de manière espacée à la paroi de puits (17).

15. Système d'aspiration (2) selon une des revendications 12 à 14, **caractérisé en ce que** le module d'aspiration (19) comprend une paroi essentiellement plate, laquelle met à disposition une surface d'étanchéité s'étendant autour du puits de réception (8) pour la mise en contact avec un joint disposé dans le domaine de la surface d'étanchéité (9) du module de cassette.

16. Système d'aspiration (2) selon une des revendications 12 à 15, **caractérisé en ce que** la module d'aspiration (19) comprend d'avantage au moins un raccord de sous-pression (24) disposé dans la paroi de puits (17), où le raccord de suppression (24), lorsque le module de cassette (1) est inséré, est situé préférablement dans le domaine de l'au moins une ouverture de vide (25), et où le raccord de sous-pression est fluidiquement en connexion avec la pompe.

17. Système d'aspiration (2) selon une des revendications 14 à 16, **caractérisé en ce que** le module de cassette (1) peut être inséré dans le module d'aspiration (19) le long d'un mouvement d'insertion s'étendant de manière rectiligne.
